# EUROPÄISCHE PATENTSCHRIFT

(11) **EP 2 205 260 B1**
(45) Veröffentlichungstag und Bekanntmachung des Hinweises auf die Patenterteilung: **18.09.2013**
(21) Anmeldenummer: 08758104.7
(22) Anmeldetag: 23.05.2008
(51) Int. Cl.: A61P 25/28, A61K 38/02, A61K 31/198

(54) **AMINOSÄURE-MINERAL-PEPTID-KOMPLEX, INSBESONDERE QUANTENMECHANISCH MODIFIZIERT, ALS ARZNEIMITTEL ZUR BEHANDLUNG VON DEMENZERKRANKUNGEN**
AMINO ACID-MINERAL-PEPTIDE COMPLEX, ESPECIALLY QUANTUM-MECHANICALLY MODIFIED, AS A MEDICAMENT FOR THE TREATMENT OF DEMENTIA DISEASES
COMPLEXE PEPTIDIQUE MINÉRAL D'ACIDES AMINÉS, EN PARTICULIER COMPLEXE MODIFIÉ PAR MÉCANIQUE QUANTIQUE, SERVANT DE MÉDICAMENT POUR TRAITER DES MALADIES DE TYPE DÉMENCE

(30) Priorität: 26.05.2007 DE 202007007542 U
(43) Veröffentlichungstag der Anmeldung: 14.07.2010
(73) Patentinhaber: Tech, Egon, 18487 Rostock (DE)
(72) Erfinder: Tech, Egon, 18487 Rostock (DE)
(74) Vertreter: Baumbach, Friedrich
(86) Internationale Anmeldenummer: PCT/DE2008/000863
(87) Internationale Veröffentlichungsnummer: WO 2008/145095

(56) Entgegenhaltungen:
- WO-A-96/30537
- WO-A-02/066043
- WO-A-2005/120487
- WO-A-2007/085238
- US-A- 5 017 375
- US-A- 5 215 969

## Beschreibung

Die Erfindung betrifft die Verwendung eines multifunktionellen Wirkstoffgemisches für die Herstellung einer pharmazeutischen Zusammensetzung zur Behandlung, Vorbeugung oder Linderung von Demenzerkränkungen, insbesondere von Morbus Alzheimer, mit präventive und therapeutischen Eigenschaften. Anwendungsgebiete der Erfindung sind die Lebenswissenschaften und die pharmazeutische Industrie.

Demenzerkränkungen sind chronische oder fortschreitende Krankheiten des Gehirns mit Störungen höherer kortikaler Funktionen, insbesondere gekennzeichnet durch Gedächtnisstörungen, Abbau des Denkvermögens, Veränderungen der Persönlichkeit und Beeinträchtigung der Selbstständigkeit im Alltag bei einer Dauer der Symptomatik von mehr als sechs Monaten. Zur Diagnostik und Differentialdiagnostik von Demenzerkrankungen werden in der Regel Eigen- und Fremdanamnese, Feststellung des neuropsychologischen Status, Feststellung des internistischen Status und Labordiagnostik verwendet. Die wörtliche Übersetzung von Demenz aus dem Lateinischen, "ohne Geist sein", verdeutlicht das Schicksal der betroffenen Personen, die die Kontrolle über ihr Denken und damit über sich selbst verlieren. Diese Veränderung der grundlegenden Wesenseigenschaften erschwert zudem den Umfang mit den Angehörigen, die durch die Entfremdung des betroffenen Familienmitglieds einer enormen Belastung ausgesetzt sind.

Charakteristisch für Demehzerkrahkungen ist die Ausbildung von Eiweißanlagerungen und/oder entzündlichen Prozessen im Gehirn, die zu Schädigungen der Nervenzellen und den damit einhergehende Symptomen führen. Für die Alzheimer Demenz sind bspw. Proteinablagerungen im Gehirn kennzeichnend, die so genannten senilen Plaques, die miteinander vernetzt und nicht wieder äbbaubar sind. Ein Bestandteil der Plaques sind die sogenannten Advanced Glycation Endproducts (AGEs), die aus Zuckerfragmentierungsprodukten gebildet werden. Die AGEs lösen entzündlicher Prozesse aus und trägen so zunächst zum Funktionsverlust, im fortgeschrittenen Stadium zur Zerstörung von Nervenzellen bei. Neuere Untersuchungen zeigen, dass bei Demenzerkrankungen die Infomationsübertragung zwischen den Nervenzellen durch Aggregation und Ablagerung von Proteinen an den Nervenenden (Synapsen) gestört ist (s. u.a. Kramer & Schulz-Schäffer, Journal of Neuroscience 2007 Feb 7;27(6):1405-10).

Bisherige medikamentöse Therapieansätze stellen im wesentlichen auf die Behandlung der Symptome von Demenzerkränkungen ab, ohne die Krankheiten ursächlich behandeln zu können. Bspw. sind Acetylcholinesterase-Hemmer die derzeit wirksamsten Medikamente gegen Morbus Alzheimer, wobei jedoch der Krahkheitsverlauf durch Eingriff in den Acetylcholin-Stoffwechsel nur durchschnittlich um neun Monate herausgezögert werden kann.

Allgemein ist benannt, dass kurze Eiweißmoleküle (Peptide) die Aggregation und Ausbildung bestimmter Protein-Plaques unterbinden können. Ferner finden Peptide für die Regulierung des menschlichen immunsystem, insbesondere als entzündungshemmende Stoffe oder Immunmodulatoren Anwendung.

Bisherige Ansätze verwenden dazu einzelne Peptide, d.h. Moleküle einer bestimmten Aminosäuresequenz, die Patienten verabreicht werden. Nächteilig daran ist, dass dadurch lediglich einzelne Vorgänge der Plaquebildung oder inflammatorischen Prozesse adressierbar sind, die den tatsächlichen komplexen Vorgängen meist nur unzureichend Rechnung tragen.

Aufgabe der Erfindung ist es daher, ein einfaches anzuwendendes und effektives Mittel für die Behandlung, Prophylaxe, und Metaphylaxe von Demenzerkrankungen bereitzustellen.

Diese Aufgabe wird durch die Verwendung eines Wirkstoffgemisches gemäß Anspruch 1 gelöst. Die weiteren Ansprüche sind Vorzugsvarianten der Erfindung.

Vollkommen überraschend hat sich die Verwendung eines multifunktionellen Wirkstoffgemisches, umfassend eine Fraktion spezifischer Peptide mit Molekulargewichten bis 10 000 Dalton und eine Fraktion essentieller und nichtessentieller Aminosäuren, als geeignet für die Herstellung einer besonders wirksamen pharmazeutischen Zusammensetzung zur Behandlung, Prophylaxe und/oder Metaphylaxe von Demenzerkrankungen erwiesen.

Erfingdungsgemäß ist dabei das multifunktionelle Wirkstoffgemisch durch Inkubation von somatischen Zellen, vorzugsweise über einen Zeitraum von 2-8 Tagen, bei geeigneten Wachstumstemperaturen, anschließende Lyse der Zellen, Ernte des Zelllysafs zusammen mit dem Erhattungsmedium, Abtrennung von Zellbestandteilen einer Molmasse über 10 000 Dalton mittels Ultrazentrifugation/Ultrafiltration, vorzugsweise bei einer Laufzeit von 12-36 h, sowie die Gewinnung der Fraktion spezifischer Peptide mit Molekulargewichten bis 10 000 Dalton und der Fraktion essentieller und nicht essentieller Aminosäuren hergestellt.

Die genaue Zusammensetzung des multifunktionellen Wirkstoffgemischs kann analytisch nicht präzise ermittelt werden, daher wird es vorliegend durch sein Herstellungsverfahren gekennzeichnet. Es enthält Polypeptide mit bis zu 80 Aminosäuren Länge, Aminosäuren sowie Salze und Mineralien. Unter Peptiden sind im Rahmen der vorliegenden Erfindung alle möglichen, sich aus mindestens zwei Aminosäureresten zusammensetzenden Peptide bzw. Peptidketten oder davon abgeleitete Strukturen zu verstehen, beispielsweise auch Peptide, die bei der Lyse somatischer Zellen freigesetzt werden. Dabei kann die Peptidfraktion Peptide verschiedenster Art umfassen, wie zum Beispiel Substanz P (SP), Delta-Schlafinduzierendes Peptid (DSIP), Vasoaktives Intestinales Peptid (VIP), Arginin-Vasotocin (AVT), Oxytocin, Samatostatin, Interleukin, Enkephaline, β-Endorphin, Neuropeptid Y, Neurotensin, Vasopressin, Corticotropin-releasing-Hormone (Corticoliberin, CRH)., Thyreotropin-releasing-Hormone (Thyreoliberin, TRH). Bei den Aminosäuren handelt es sich um alle essentiellen und nichtessentiellen Aminosäuren, insbesondere um Glycin. Des Weiteren umfasst die Fraktion Mineralien und Salze der Elemente Magnesium, Calcium, Kalium, Zink, Lithium, Phosphor, Silizium, Mangan, Chrom, Selen bzw. Molybdän.

Durch die herstellungsbedingte Kombination unterschiedlicher Peptide mit verschiedenen Eigenschaften wird erstmalig ein Wirkmechanismus ermöglicht, der mit den herkömmlichen Peptiden bislang nicht beobachtet werden konnte. Vorteilhaft ist ferner, dass dieses neuartige Wirkstoffgemisch zu einem wesentlichen Anteil Peptide und Aminosäuren enthält, die geeignet sind, die Blut-Hirn-Schranke zu überwinden. Es hat sich daher in der Praxis bei unterschiedlichen neurodegenerativen Erkrankungen bewährt. Insbesondere weisen die Erkenntnisse darauf hin, dass durch die in dem Wirkstoffgemisch enthaltenen Peptide die schädigende Aggregation von Peptiden und Proteinen und die damit verbundene Ausbildung von Eiweißablagerungen im menschlichen Gehirn wenigstens vermindert wird sowie die entzündlichen Prozessen durch immunmodulierende Wirkung zumindest gehemmt werden.

Der Vorteil des erfindungsgemäßen Wirkstoffgemischs besteht ferner darin, dass nach Absättigung der körpereigenen Bindungsstellen mit Wirkstoffsubstanz keine weitere Anreicherung der Wirkstoffsubstanz am Zielort erfolgt, sondern die Wirkstoffsubstanz rasch aus dem Körper ausgeschieden wird und somit Überdosierungen vermieden werden.

Besonders geeignet ist das erfindungsgemäße Wirkstoffgemisch für die Behandlung, Prophylaxe und/oder Metaphylaxe von Demenzerkrankungen gemäß dem ICD(International Classification of Diseases and Related Health Problems)-10-Code F00=F03, insbesondere von Krankheiten des ICD-10-Code F00 und F02.

Die Erfindung basiert also auf der Erkenntnis, dass ein bestimmtes multifunktionelles Wirkstoffgemisch durch Inhibierung von Eiweißablagerungen und gleichzeitige Immunmnodulation zur Behandlung, Vorbeugung oder Linderung von unterschiedlichen Demenzerkrankungen geeignet ist.

Vorzugsweise umfasst die Erfindung die Verwendung des hier beschriebenen Wirkstoffgemisches zur Herstellung pharmazeutischer Zusammensetzungen zur Behandlung, Prophylaxe und/oder Metaphylaxe von wenigstens einer medizinischen Indikation aus der Liste: Morbus Alzheimer, frontotemporale Demenz, Morbus Pick, Lewy-Körperchen-Erkrankung, Morbus Parkinson, Lewy-Body-Demenz, Creutzfeldt-Jakob-Krankheit und/oder Chorea Huntington. Vorteilhafterweise ermöglicht die Erfindung die Herstellung einer pharmazeutischen Zusammensetzung, die gleichzeitig für die Behandlung, Prophylaxe und/oder Metaphylaxe von mehreren oder allen, vorzugsweise wenigstens zwei, bevorzugt mindestens drei, insbesondere bevorzugt wenigstens vier, dieser Krankheiten geeignet ist. Da die Symptome von Demenzerkrankungen in ihren frühen Stadien oftmals ähnlich sind, erlaubt die erfindungsgemäße pharmazeutische Zusammensetzung eine frühzeitige Behandlung und Prophylaxe unterschiedlicher Demenzerkrankungen, bevor sich die typischen Symptome manifestiert haben.

In einer besonders geeigneten Ausführungsform erfolgt die Verwendung des Wirkstoffgemisches für die Herstellung einer pharmazeutischen Zusammensetzung zur Behandlung, Prophylaxe und/oder Metaphylaxe von Morbus Alzheimer und/oder frontotemporaler Demenz, insbesondere von Morbus Alzheimer und frontotemporaler Demenz, besonders bevorzugt von Morbus Alzheimer und Morbus Pick.

In einer weiteren günstigen Ausgestaltung wird das erfindungsgemäße Wirkstoffgemisch für die Herstellung einer pharmazeutischen Zusammensetzung zur Behandlung, Prophylaxe und/oder Metaphylaxe der Lewy-Körperchen-Erkankung und/oder von Morbus Parkinson, verwendet, insbesondere der Lewy-Körperchen-Erkrankung und von Morbus Parkinson, besonders bevorzugt der Lewy-Body-Demenz.

In einer anderen vorteilhaften Ausführungsform wird das erfindungsgemäße Wirkstoffgemisch für die Herstellung einer pharmazeutischen Zusammensetzung zur Behandlung, Prophylaxe und/oder Metaphylaxe der Creutzfeldt-Jakob-Krankheit oder von Chorea Huntington verwendet.

Bevorzugt wird eine pharmazeutisch geeignete Menge des multifunktionellan Wirkstoffgemisches für die erfindungsgemäße Verwendung gewählt, dem vorzugsweise ein pharmazeutisch annehmbarer Carrier und/oder Verdünner und/oder Exzipient zugesetzt ist.

Ausgangsmaterial für die Peptid- und Aminosäurefraktion des multifunktionellen Wirkstoffgemischs sind somatische Zellen. Geeignet sind etwa Zellen der diploiden Kälbemieren-Zelllinie MDBK, Hühnerfibroblasten oder Zellen einer aus Schweinen stammenden diploiden Tubenepithel-Zelllinie. Für die Herstellung patientenspezfischer Wirkstoffe können beispielsweise auch körpereigene Zellen verwendet werden, die aus Körperflüssigkeiten oder Gewebe gewonnen wurden, insbesondere auch solche, die aus erkranktem Gewebe oder Körperflüssigkeiten gewonnen wurden, wodurch die erfindungsgemäße Zusammensetzung besonders spezifisch und verträglich ist.

Die Zellen werden bei den für die Ursprungsspezies typischen Temperaturen von 20°C bis 39°C über einen Zeitraum von 2 - 8 Tagen inkubiert, anschließend lysiert und mit dem Erhaltungsmedium geerntet.

Zur Entfernung von Schwebeteilchen kann das Zelllysat anschließend durch einen Filter mit der Porengröße 0,65 µm filtriert werden. Eine Zusätzliche optionale Filtration durch einen Filter der Porengröße 0,1 µm erhöht die Effizienz der folgenden Ultrazentrifugation. Das so gewonnene Permeat wird anschließend einer Ultrazentrifugation über einem Filtrationsmodul mit einer Ausschlußgröße kleiner als 10 000 Dalton unterzogen. Die Ultrazentrifugation hat vorzugsweise eine Laufzeit von etwa 24 Stunden. Auf diese Weise wird ein Filtrat gewonnen, das nur Substanzen enthält, die kleiner als 10 000 Dalton sind und damit auch das Gemisch spezifischer Peptide mit Molekulargewichten bis 10 000 Dalton und/oder die Fraktion mit essentiellen und nichtessentiellen Aminosäuren. Aber auch Filter mit anderen Porengrößen sind geeignet, wobei die Porengröße so ausgewählt wird, dass das Wirkstoffgemisch die gewünschte maximale Molekül- oder Molekülaggregatgrößen von bis zu 10 000 Dalton aufweist.

Der wesentliche Vorteil der Ultrazentrifugation ist dabei, dass bei diesem Prozess auch eine lokale Aufkonzentration des Wirkstoffgemischs innerhalb des zentrifugierten Mediums erfolgt. Weiterhin bietet die Ultrazentrifugation durch die definierte räumliche Verteilung der Wirkstoffe im zentrifugierten Medium, die meist abhängig vom Molekulargewicht oder der räumlichen Struktur der zentrifugierten Bestandteile ist, die Möglichkeit, auch gezielt Fraktionen mit einer gewünschten Bandbreite an Molekulargewichten öder räumlichen Strukturen aus dem Medium zu entnehmen.

Bevorzugt wird ein multifunktionelles Wirkstoffgemisch einer Fraktion spezifischer Peptide mit einer relativen Molmasse zwischen 200 bis 6.000 Dalton eingesetzt. Dieses Gemisch wird durch Aufkonzentration um den Faktor 1000 der gewonnenen Fraktion mit Proteinen kleiner als 10 000 Dalton mit physikalischen Mitteln erhalten.

Insbesondere die Dosis-Wirkungsbeziehung des erfindungsgemäßen Wirkstoffgemisches nach dem Prinzip der Hyperbelkurve ermöglicht die spezifische Inhibierung der schädlichen Interaktion von einem oder mehreren Substanzen des Impfstoffs mit körpereigenen Stoffen bzw. Komponenten des Immunsystems. Der erfindungsgemäße Vorteil im Vergleich zu einer linearen Dosis-Wirkungsbeziehung besteht darin, dass nach Absättigung der körpereigenen Bindungsstellen mit Wirkstoffsubstanz keine weitere Anreicherung der Wirkstoffsubstanz am Zielort erfolgt, sondern die Wirkstoffsubstanz rasch aus dem Körper ausgeschieden wird und somit Überdosierungen auch bei hohen Konzentrationen vermieden werden.

Als Applikationsform sind alle gängigen Applikationsformen für Wirkstoffe, insbesondere solche für pharmazeutisch wirksame Peptide und/oder Proteine geeignet, vorzugsweise per os, bspw. als Tablette oder Saft, oder bevorzugt über die Haut, bspw. als Salbe oder Gel. Vorrangig kommt die intranasale und sublinguale Form in Frage, eine intramuskuläre Applikation, beispielsweise durch eine Injektion ist auch geeignet.

Um den Vorteil einer spezifischen Mischung kleiner 10kDa zu nutzen, ist die intranasale Form bevorzugt geeignet, die eine schnelle, wirkungsvolle und einfache Applikation ermöglicht.

Neben dem eigentlichen Wirkstoff hat insbesondere das Verabreichungsverfahren einen wesentlichen Einfluss auf die Wirksamkeit pharmazeutischer Zusammensetzungen. Im Rahmen der vorliegenden Erfindung sind auch neuartige Verabreichungsverfahren für die erfindungsgemäße Zusammensetzung besonders geeignet. Diese neuen Verabreichungsverfahren umfassen insbesondere die Methode der AKUJEKTUR®.

Weiterentwicklungen aus dem Bereich der Nanotechnologie und der angewandten Quantenphysik eröffnen die Möglichkeit zur Herstellung eines neuen Spektrums von Wirkstoffgemischen. Derartige Wirkstöffgemische werden in einer besonders geeigneten Ausgestaltung der Erfindung für die Herstellung einer pharmazeutischen Zusammensetzung zur Behandlung, Prophylaxe und/oder Metaphylaxe von Demenzerkrankungen eingesetzt.

Die Aufgabe der vorliegenden Erfindung bestand demnach auch darin, neue multifunktionelle Wirkstoffgemische, unter Einsatz von Verfahren bereitzustellen, die neueste Erkenntnisse physikalischer Phänomene der Quantensteuerung, verbunden mit Verhaltensweisen von Biologicals und Minerals und deren gewählter Komplexe im EMF (Elektro Magnetischen Feld) und deren neuartige, bisher nicht bekannt Wirkungsweisen auf den Organismus ausnutzen, um Verfahren zur Herstellung effizienter pharmazeutischer Zusammensetzungen für die Behandlung, Prophylaxe und/oder Metaphylaxe von Demenzerkrankungen anzugeben. Somit handelt es sich bei dem multifunktionellen Wirkstoffgemisch um einen Aminosäure-Mineral-Peptid-Komplex, der quantenmechanisch modifiziert ist (AMIPEC-Q).

In einer weiteren Ausführungsform umfasst das multifunktionelle Wirkstoffgemisch daher ferner energetisierte Silizium-Nanopartikel, Zeolithe, Bentonit, Montmorillonite oder andere mineralische Stoffe oder Stoffgemische, die zur Optimierung der Steuerungsprozesse pflanzlicher oder tierischer Zellen, Zellsysteme, Organe oder Organismen mit Informationen beladen wurden indem sie etwa 2 bis 5 Minuten einer Mikrowellenstrahlung von 2,45 Gigahertz ausgesetzt wurden, die eine Veränderung der Kristallstrukturen der mineralischen Stoffe herbeiführt.

Das Verfahren zur Herstellung der energetisierten Silizium-Nanopartikel, Zeolithe, Bentonite, Montmorillonite oder anderer mineralischer Stoffe oder Stdffgemische im erfindungsgemäßen Sinn, für die Herstellung des Wirkstoffgemischs, soll nachfolgend an einem Beispiel erläutert werden.

Verwendet wurden sowohl Zeolithe mit einem Durchmesser kleiner als 100 Mikrometer als auch fertige Nano- und Mikropartikel mit SiO₂ der Produktreihe Köstrosol (Schutzmarke) des Chemiewerkes Köstritz.

Die verwendeten Partikel wurden durch den Einsatz eines Magnetrons mit der Frequenz von 2450 Megahertz unterschiedlichen Dosen/Impulsen von Mikrowellen ausgesetzt, was zu Änderungen im Kristallgitter und zur Aufnahme von Informationen über die Mikrowellen führt, die je nach Zielort und Indikationsstellung variiert werden können. Eine der physiologischsten Impulsfrequenzen kristallisierte sich heraus, es ist die Schumann-Frequenz mit 7,83 Hertz. Andere sind aber auch möglich und von der Indikationsstellung abhängig, alle Möglichkeiten gehören zum Umfang der vorliegenden Erfindung.

Bevorzugt wird ein spezielles Köstrosol mit einem Partikeldurchmesser von 7 Nanometern verwendet, das in der Lage ist, Stoffe mit ähnlicher Partikelgröße locker zu binden und als Transporter für die bevorzugten Stoffe zum Zielort zu dienen. Am Zielort kommt es zur Übermittlung der applizierten Schwingung durch die Partikel. Die Zelle-/Zellsysteine nehmen diese über die Gel-Sol-Formulierung auf und bekommen ädhäsiert oder inkludiert das beschriebene Wirkstoffgemisch bzw. deren Fraktionen, bzw. die aufgeladene Aminosäure Glycin, die dann ähnlich der Wirkungsweise der Substanz P (Hecht/Oehme et al.) wirkt, appliziert.

Das Zusammenspiel von mineralischen, aufgeladenen Partikeln und die Veränderung des Verhaltens der organischen Bestandteile, wie sie in der vorliegenden Erfindung aufgeführt sind, insbesondere das veränderte Verhalten der Aminosäure Glycin (Neurotransmitter), lässt völlig neue Indikationsstellungen zu. Die mineralische Komponente dient hierbei als Daten- und Informationsträger verschiedener Schwingungsgrößen, die in einer Gel-Sol-Formulierung (wie alle Körperflüssigkeiten) durch die organischen Komponenten im Nano- und Mikrobereich an Zellen und Zellverbände vermittelt werden.

Die Herstellung ist äußerst kostengünstig und mögliche Nebenwirkurigen können weitestgehend ausgeschlossen werden.

Alle in der vorangehenden Beschreibung und den nachfolgenden Ansprüchen dargestellten Merkmale können sowohl einzeln als auch in beliebiger Kombination für die Verwirklichung der Erfindung in ihren verschiedenen Ausgestaltungen von Bedeutung sein.

## Patentansprüche

1. Verwendung eines multifunktionellen Wirkstoffgemisches, umfassend eine Fraktion spezifischer Peptide mit Molekulargewichten bis 10 000 Dalton und eine Fraktion essentieller und nichtessentieller Aminosäuren, gewonnen durch
- Inkubation von somatischen Zellen bei geeigneten Wachstumstemperaturen,
- anschließende Lyse,
- Ernte zusammen mit dem Erhaltungsmedium und
- Abtrennung von Zellbestandteilen, die eine Molmasse über 10 000 Dalton aufweisen, mittels Ultrazentrifugation/Ultrafiltration und
- Gewinnung der Fraktion spezifischer Peptide mit Molekulargewichten bis 10 000 Dalton und der Fraktion essentieller und nicht essentieller Aminosäuren,
für die Herstellung einer pharmazeutischen Zusammensetzung zur Behandlung, Prophylaxe und/oder Metaphylaxe von Demenzerkrankungen.

2. Verwendung nach Anspruch 1 für die Herstellung einer pharmazeutischen Zusammensetzung zur Behandlung, Prophylaxe und/oder Metaphylaxe von Krankheiten nach dem ICD-10-Code F00, F01, F02 und/oder F03, vorzugsweise für die Herstellung einer pharmazeutischen Zusammensetzung zur Behandlung, Prophylaxe und/oder Metaphylaxe von Krankheiten nach dem ICD-10-Code F00 und/oder F03.

3. Verwendung nach einem der vorangehenden Ansprüche für die Herstellung einer pharmazeutischen Zusammensetzung zur Behandlung, Prophylaxe und/oder Metaphylaxe von
Morbus Alzheimer, frontotemporaler Demenz, Morbus Pick, der Lewy-Körperchen-Erkrankung, Morbus Parkinson, Lewy-Body-Demenz, der Creutzfeldt-Jakob-Krankheit und/oder Chorea Huntington.

4. Verwendung nach einem der vorangehenden Ansprüche für die Herstellung einer pharmazeutischen Zusammensetzung zur Behandlung, Prophylaxe und/oder Metaphylaxe von Morbus Alzheimer und/oder frontotemporaler Demenz, vorzugsweise für die Herstellung einer pharmazeutischen Zusammensetzung zur Behandlung, Prophylaxe und/oder Metaphylaxe von Morbus Alzheimer und Morbus Pick.

5. Verwendung nach einem der Ansprüche 1-3 für die Herstellung einer pharmazeutischen Zusammensetzung zur Behandlung, Prophylaxe und/oder Metaphylaxe der Lewy-Körperchen-Erkrankung und/oder von Morbus Parkinson.

6. Verwendung nach einem der Ansprüche 1-3 oder 5 für die Herstellung einer pharmazeutischen Zusammensetzung zur Behandlung, Prophylaxe und/oder Metaphylaxe der Lewy-Body-Demenz.

7. Verwendung nach einem der Ansprüche 1-3 für die Herstellung einer pharmazeutischen Zusammensetzung zur Behandlung, Prophylaxe und/oder Metaphylaxe der Creutzfeldt-Jakob-Krankheit oder
für die Herstellung einer pharmazeutischen Zusammensetzung zur Behandlung, Prophylaxe und/oder Metaphylaxe von Chorea Huntington.

8. Verwendung nach einem der vorangehenden Ansprüche, wobei das multifunktionelle Wirkstoffgemisch Mineralien und/oder Salze umfasst.

9. Verwendung nach einem der vorangehenden Ansprüche, wobei das multifunktionelle Wirkstoffgemisch Mineralien und/oder Salze der Elemente Magnesium, Calcium, Kalium, Zink, Lithium, Phosphor, Silizium, Mangan, Chrom, Selen und/oder Molybdän umfasst.

10. Verwendung nach einem der vorangehenden Ansprüche, wobei dem multifunktionellen Wirkstoffgemisch wenigstens ein pharmazeutisch annehmbarer Carrier und/oder Verdünner und/oder Exzipient zugesetzt ist.

11. Verwendung nach einem der vorangehenden Ansprüche, wobei dem multifunktionellen Wirkstoffgemisch energetisierte Silizium-Nanopartikel, Zeolithe und/oder Bentonite, Montmorillonite oder andere mineralische Stoffe und/oder Stoffgemische, die zur Optimierung der Steuerungsprozesse pflanzlicher oder tierischer Zellen, Zellsysteme, Organe, oder Organismen mit Informationen beladen wurden indem sie Mikrowellenstrahlung einer bestimmten Dosis und Frequenz ausgesetzt werden,
zugesetzt sind.

12. Verwendung nach einem der vorangehenden Ansprüche, wobei die Inkubation von Zellen bei geeigneten Wachstumstemperaturen über einen Zeitraum von 2 - 8 Tagen erfolgt.

13. Verwendung nach einem der vorangehenden Ansprüche, wobei die Abtrennung mittels Ultrazentrifugation/Ultrafiltration mit einem Filter und/oder bei einer Laufzeit von 24 Stunden erfolgt.

14. Verwendung nach einem der vorangehenden Ansprüche, wobei eine Fraktion spezifischer Peptide mit Molekulargewichten zwischen 200 und 6000 Dalton und/oder eine Fraktion mit essentiellen und nicht essentiellen Aminosäuren eingesetzt wird.

15. Verwendung nach einem der vorangehenden Ansprüche, wobei die pharmazeutische Zusammensetzung zur Verabreichung mittels Akujektur geeignet ist.

## Claims

1. Use of a multifunctional active agent mixture, comprising a fraction of specific peptides with molecular weights up to 10,000 Daltons and a fraction of essential and non-essential amino acids, obtained by
- incubating somatic cells at appropriate growth temperatures
- subsequent lysis
- harvesting together with the growth medium
- separating cellular components which have molecular weights greater than 10,000 Daltons via ultracentrifugation / ultrafiltration
- obtaining the fraction of specific peptides having molecular weights up to 10,000 Dalton and the fraction of essential and non-essential amino acids,
for the production of a pharmaceutical composition for the treatment, prophylaxis and/or metaphylaxis of dementia.

2. Use according to claim 1 for the production of a pharmaceutical composition for the treatment, prophylaxis and/or metaphylaxis of diseases according to the ICD-10-Code F00, F01, F02 and/or F03, preferably for the treatment, prophylaxis and/or metaphylaxis of diseases according to the ICD-10-Code F00 and/or F03.

3. Use according to any one of the previous claims for the production of a pharmaceutical composition for the treatment, prophylaxis and/or metaphylaxis of Alzheimer's disease, frontotemporal dementia, Pick's disease, Lewy body disease, Parkinson's disease, Lewy body dementia, Creutzfeld-Jakob disease and/or Huntington's disease.

4. Use according to any one of the previous claims for the production of a pharmaceutical composition for the treatment, prophylaxis and/or metaphylaxis of Alzheimer, disease and/or frontotemporal dementia, preferably for the production of a pharmaceutical composition for the treatment, prophylaxis and/or metaphylaxis of Alzheimer's disease and Pick's disease.

5. Use according to any one of the claims 1-3 for the production of a pharmaceutical composition for the treatment, prophylaxis and/or metaphylaxis of Lewy body dementia and/or Parkinson's disease.

6. Use according to any one of the claims 1-3 or 5 for the production of a pharmaceutical composition for the treatment, prophylaxis and/or metaphylaxis of Lewy body dementia.

7. Use according to any one of the claims 1-3 for the production of a pharmaceutical composition for the treatment, prophylaxis and/or metaphylaxis of the Creutzfeld-Jakob disease or for the production of a pharmaceutical composition for the treatment, prophylaxis and/or metaphylaxis of the Huntington's disease.

8. Use according to any one of the previous claims, **characterized in that** the multifunctional active agent mixture comprises minerals and/or salts.

9. Use according to any one of the previous claims, **characterized in that** the multifunctional active agent mixture comprises minerals and/or salts of the elements magnesium, calcium, potassium, zinc, lithium, phosphor, silicium, manganese, chrome, selenium and/or molybdenum.

10. Use according to any one of the previous claims, **characterized in that** at least one pharmaceutically acceptable carrier and/or thinner and/or excipient is added to the multifunctional active agent mixture.

11. Use according to any one of the previous claims, **characterized in that** energised silicium-nanoparticles, zeolite and/or bentonite, montmorillonite or other mineral substances and/or mixture of substances, which are loaded with information for the optimization of regulatory processes in plant or animal cells, cell systems, organs, or organisms with **in that** they were exposed to microwave radiation of a certain dose and frequency, are added to the multifunctional active agent mixture.

12. Use according to any one of the previous claims, **characterized in that** the incubation of the cells at appropriate temperatures occurs over a period of 2-8 days.

13. Use according to any one of the previous claims, **characterized in that** the separation via ultracentrifugation / ultrafiltration is carried out with a filter and/or over a runtime of 24 hours.

14. Use according to any one of the previous claims, **characterized in that** a fraction of specific peptides with molecular weights between 200 and 6000 Daltons and/or a fraction of essential and non-essential amino acids are used.

15. Use according to any one of the previous claims, **characterized in that** the pharmaceutical composition is suitable for the administration via akujektur.

## Revendications

1. Utilisation d'un mélange d'agents actifs multifonctionnel qui se compose d'une fraction de peptides spécifiques de poids moléculaires allant jusqu'à 10 000 daltons et d'une fraction contenant des acides aminés essentiels et non-essentiels, et qui a été extrait par
- incubation de cellules somatiques à des températures de croissance appropriées,
- suivie d'une lyse,
- d'une récolte en même temps que le milieu de conservation et
- d'une séparation par ultracentrifugation/ultrafiltration d'éléments cellulaires qui présentent un poids moléculaire de plus de 10 000 daltons, et
- d'une extraction de la fraction de peptides spécifiques de poids moléculaires allant jusqu'à 10 000 daltons et de la fraction contenant des acides aminés essentiels et non-essentiels,
pour la préparation d'une composition pharmaceutique destinée au traitement, à la prophylaxie ou à la métaphylaxie des démences.

2. Utilisation suivant la revendication 1 pour la préparation d'une composition pharmaceutique
destinée au traitement, à la prophylaxie ou à la métaphylaxie de maladies appartenant aux catégories F00, F01, F02 ou F03 de la CIM 10, de préférence pour la préparation d'une composition pharmaceutique destinée au traitement, à la prophylaxie ou à la métaphylaxie des maladies appartenant aux catégories F00 ou F03 de la CIM 10.

3. Utilisation selon l'une quelconque des revendications précédentes pour la préparation d'une composition pharmaceutique destinée au traitement, à la prophylaxie ou à la métaphylaxie de la maladie d'Alzheimer, de la démence frontotemporale, de la maladie de Pick, de la démence à corps de Lewy[CB1], de la maladie de Parkinson, de la maladie de Creutzfeldt-Jakob ou de la démence de la chorée de Huntington.

4. Utilisation selon l'une quelconque des revendications précédentes pour la préparation d'une composition pharmaceutique destinée au traitement, à la prophylaxie ou à la métaphylaxie de la maladie d'Alzheimer ou de la démence frontotemporale, de préférence pour la préparation d'une composition pharmaceutique destinée au traitement, à la prophylaxie ou à la métaphylaxie de la maladie d'Alzheimer et de la maladie de Pick.

5. Utilisation selon l'une quelconque des revendications 1 à 3, pour la préparation d'une composition pharmaceutique destinée au traitement, à la prophylaxie ou à la métaphylaxie de la démence à corps de Lewy ou de la maladie de Parkinson.

6. Utilisation selon l'une quelconque des revendications 1 à 3 ou 5, pour la préparation d'une composition pharmaceutique destinée au traitement, à la prophylaxie ou à la métaphylaxie de la démence à corps de Lewy.

7. Utilisation selon l'une quelconque des revendications 1 à 3, pour la préparation d'une composition pharmaceutique destinée au traitement, à la prophylaxie ou à la métaphylaxie de la maladie de Creutzfeldt-Jakob ou pour la préparation d'une composition pharmaceutique destinée au traitement, à la prophylaxie ou à la métaphylaxie de la démence de la chorée de Huntington.

8. Utilisation selon l'une quelconque des revendications précédentes **caractérisée en ce que** le mélange d'agents actifs multifonctionnel contient des minéraux ou des sels.

9. Utilisation selon l'une quelconque des revendications précédentes **caractérisée en ce que** le mélange d'agents actifs multifonctionnel contient des minéraux ou des sels d'éléments comme le magnésium, le calcium, le potassium, le zinc, le lithium, le phosphore, le silicium, le manganèse, le chrome, le sélénium ou le molybdène.

10. Utilisation selon l'une quelconque des revendications précédentes **caractérisée en ce que** le mélange d'agents actifs multifonctionnel est ajouté à au moins un vecteur, diluant ou excipient acceptable sur le plan pharmaceutique.

11. Utilisation selon l'une quelconque des revendications précédentes **caractérisée par** l'addition au mélange d'agents actifs multifonctionnel de nanoparticules de silicium énergisées, de zéolithe ou de bentonite, de montmorillonite ou d'autres substances ou mélanges de substances minérales qui ont été chargés d'informations permettant d'optimiser les processus de gestion des cellules, groupes de cellules, organes ou organismes végétaux ou animaux, en les exposant à une dose précise de rayonnement micro-ondes de fréquence déterminée.

12. Utilisation selon l'une quelconque des revendications précédentes **caractérisée en ce que** l'incubation des cellules s'effectue à des températures de croissance appropriées sur une période de 2 à 8 jours.

13. Utilisation selon l'une quelconque des revendications précédentes **caractérisée en ce que** la séparation s'effectue par ultracentrifugation/ultrafiltration à l'aide d'un filtre ou sur une période de 24 heures.

14. Utilisation selon l'une quelconque des revendications précédentes **caractérisée par** l'utilisation d'une fraction de peptides spécifiques de poids moléculaires compris entre 200 et 6 000 daltons ou une fraction contenant des acides aminés essentiels et non-essentiels.

15. Utilisation selon l'une quelconque des revendications précédentes **caractérisée en ce que** la composition pharmaceutique est appropriée à l'administration au moyen d'Akujektur.
